# FASCICULE DE BREVET EUROPEEN

(11) **EP 0 585 455 B1**
(45) Date de publication et mention de la délivrance du brevet: **03.09.1997**
(21) Numéro de dépôt: 93920538.1
(22) Date de dépôt: 19.03.1993
(51) Int. Cl.: A61B 17/60

(54) **APPAREIL ORTHOPEDIQUE LINEAIRE DE FIXATION EXTERNE**
LINEARE ORTHOPAEDISCHE VORRICHTUNG FUER EXTERNE FIXATION
LINEAR ORTHOPEDIC DEVICE FOR EXTERNAL SETTING OF FRACTURES

(30) Priorité: 19.03.1992 FR 9203275
(43) Date de publication de la demande: 09.03.1994
(73) Titulaire: LONIEWSKI, Xavier, F-51022 Chalons-sur-Marne (FR)
(72) Inventeur: LONIEWSKI, Xavier, F-51022 Chalons-sur-Marne (FR)
(74) Mandataire: L'Helgoualch, Jean
(86) Numéro de dépôt international: FR9300278
(87) Numéro de publication internationale: WO9318717

(56) Documents cités:
- EP-A- 0 140 786
- EP-A- 0 240 034
- BE-A- 389 476
- DE-A- 3 515 678
- FR-A- 1 206 411
- FR-A- 2 405 063
- FR-A- 2 499 400
- FR-A- 2 547 676

## Description

La présente invention concerne un fixateur externe linéaire destiné à la chirurgie orthopédique afin de réduire une fracture et immobiliser un os fracturé.

L'ostéosynthèse par fixateur externe s'est largement développée depuis une dizaine d'années, notamment pour la chirurgie de guerre. Son emploi est nécessaire pour les fractures ouvertes ou complexes des membres, particulièrement à chaque fois qu'une ostéosynthèse intra focale est contre-indiquée.

Le fixateur proposé par l'invention est linéaire et compatible avec certains fixateurs linéaires ou circulaires employés en chirurgie orthopédique et traumatologique notamment de guerre. Il est prévu pour être utilisé par une antenne chirurgicale mobile.

La première, qualité que doit présenter un fixateur est la rigidité mécanique. En effet, son rôle étant de favoriser la consolidation des fragments osseux d'une fracture, il doit assurer l'immobilisation stricte de ceux-ci.

Les fixateurs doivent permettre une intervention rapide par les chirurgiens et pour ce faire, leur manipulation doit être facile dans tous les cas de fractures.

Outre la rigidité et la simplicité de montage, ils ne doivent pas avoir un poids trop important afin d'en augmenter la tolérance à long terme par les patients.

De plus, la radiotransparence et l'amagnétisme peuvent considérablement faciliter la surveillance de la consolidation osseuse.

Les fixateurs utilisés jusqu'ici ne satisfont pas à l'ensemble de ces critères.

On connait des fixateurs externes linéaires se composant d'un tube métallique rigide, présentant dans deux plans perpendiculaires des orifices diamétralement opposés régulièrement espacés et filetés, dans lesquels les fiches sont engagées et bloquées par des vis.

Le brevet FR 2457676 décrit un fixateur linéaire permettant de réduire et contenir une fracture. Il comprend un support en forme de tube portant deux segments de tubes présentant dans deux plans perpendiculaires entre eux des orifices diamétralement opposés régulièrement espacés et filetés destinés aux broches ou fiches. L'assemblage des segments de tubes avec le support est réalisé à l'aide de rotules sphériques. Ces rotules sont prolongées par des colliers dont le coulissement sur le support entraîne le déplacement des deux segments de tubes perforés pour permettre la réduction et la contention de la fracture. Ces dispositifs conçus pour être portés par des patients qui sont en mouvement présentent plusieurs inconvénients. Les colliers à rotule qui serrent et bloquent les broches engagées dans l'os ont tendance à se desserrer pendant les mouvements de marche des patients et donc n'assurent pas une bonne réduction et immobilisation de l'os pendant la consolidation.

Le deuxième reproche que l'on peut faire à ces dispositifs c'est qu'ils sont lourds du fait qu'ils sont en métal et présentent, pour la même raison, l'inconvénient d'être radio opaques.

Troisièmement le dispositif nécessite cinq constituants de base : support, tube, rotule, collier, broche ou fiche ; il ne permet pas une manipulation facile, du fait du nombre élevé de ses constituants de base.

Il existe des dispositifs orthopédiques en matériau radiotransparent plus légers que les précédents, tel le fixateur décrit par le brevet FR 2 405 063. Ce fixateur comporte des supports ou tiges de liaison qui permettent d'assembler les pièces portant les broches par l'intermédiaire de rotules et de colliers. Il doit encadrer complétement le membre blessé, et de ce fait n'a pas la souplesse d'utilisation des fixateurs linéaires, dont le système de base est une tige ou poutre parallèle à l'os. Ces tiges ou poutres peuvent être assemblées en échafaudage et orientées dans toutes les directions l'une par rapport à l'autre pour permettre le traitement des cas les plus compliqués, comme les fractures comminutives.

De plus, le fixateur décrit dans ce brevet présente un nombre de constituants de base, encore trop grand.

Le but de l'invention est donc de proposer un fixateur linéaire possédant les caractéristiques équivalentes du fixateur connu et décrit par le brevet FR 2457676 sans ses inconvénients. Il a une meilleure rigidité car il assure un serrage des fiches sur les tiges plus performant, donc une meilleure immobilisation de l'os. Il est plus léger et radiotransparent. Outre, la radiotransparence, il est amagnétique et non conducteur d'électricité. Il présente une simplicité de montage qui le rend adapté à la chirurgie de guerre ou de catastrophe. Il est donc conçu pour faciliter la manipulation par le chirurgien et permettre la surveillance de la consolidation de l'os.

De plus, le fixateur est équipé de fiches autoforeuses et autotaraudeuses de différents diamètres adaptés au site d'implantation.

L'invention a donc pour objet un appareil orthopédique linéaire de fixation externe des fractures, comportant au moins une tige rigide pleine présentant des orifices transversaux pour le passage des fiches vissées dans l'os de part et d'autre du foyer de fracture et fixée solidement aux fiches par des moyens qui les solidarisent.

Cette structure présente l'avantage d'avoir une très grande rigidité du fait que la tige est pleine et de mettre en jeu un nombre de constituants de base réduits à trois : tige, fiche, moyen pour les solidariser.

La tige portant les fiches est ici affranchie d'un support, contrairement aux systèmes décrits dans les deux brevets cités plus haut.

L'invention a également pour objet un fixateur tel que défini ci-dessus, caractérisé de plus en ce que le matériau constitutif des tiges, fiches et de certains éléments des moyens qui les solidarisent est radio-transparent, amagnétique et non conducteur d'électricité. La consolidation de l'os peut donc être suivie par radiographie, résonance magnétique nucléaire. Les explorations électrophysiologiques sont aussi facilitées.

L'utilisation d'un tel matériau permet d'avoir un fixateur plus léger, et donc plus adapté au port permanent par les patients.

Dans un premier mode de réalisation de l'invention, les moyens qui solidarisent la fiche avec la tige sont des colliers de serrage munis de moyens de blocage. Dans un autre mode de réalisation préférentiel de l'invention, les moyens qui solidarisent la fiche avec la tige sont des inserts de fixation, afin de minimiser l'encombrement. Ces inserts ont des diamètres variables, suivant le type de fiche utilisée. Dans une variante de réalisation, la tige rigide pleine est constituée de deux demi-tiges rigides pleines longitudinales, présentant des crans pour le passage des fiches et assemblées par des colliers de serrage munis de moyens de blocage.

Cette variante a l'avantage de permette de réaliser des montages plus complexes, ou de reprendre des montages pour défaut de réduction car elle offre la possibilité de conserver l'implantation initiale des fiches dans l'os, ce qui est un point important pour des patients dont le capital osseux ou les sites d'implantation des fiches peuvent être limités par les dégâts occasionnés par le traumatisme.

Un autre avantage de l'invention réside dans sa très grande souplesse d'emploi et la possibilité de modifier aisément la géométrie du montage. L'invention est adaptée à tous les cas de fractures, ce qui rend son emploi universel.

Les colliers de serrage sont encliquetables pour une meilleure tenue du système et assurent une plus grande sécurité au patient. Ils sont munis de boulons freinés à blocage automatique, dans ce même but.

Dans une variante de réalisation, le fixateur est caractérisé en ce qu'il comporte plusieurs tiges assemblées dans un montage complexe ou "échafaudage" grâce à des colliers associés deux à deux et bloqués de la même manière que précédemment.

Dans un mode d'utilisation de l'invention, qui dépendra du type de fracture à traiter, il sera nécessaire d'incliner ou de déporter la fiche par rapport à l'axe perpendiculaire à la tige et de fixer celle-ci à la tige par un collier porte-fiche. Le fixateur selon l'invention peut être muni de fiches de diamètre adapté qui sont à la fois autoforeuses et autotaraudeuses. Ainsi, il n'y a pas de forage préalable de l'os par une mèche, les fiches pouvant à la fois réaliser la perforation et le taraudage de la structure osseuse.

D'autres avantages apparaîtront au cours de la description suivante de modes de réalisation du fixateur selon l'invention,non limitative et en référence aux dessins annexés, parmi lesquels :
La figure 1 représente une vue en coupe d'une tige pleine présentant des orifices transversaux.
La figure 2 est une vue de profil de la même tige équipée de fiches et de colliers.
La figure 3 représente un premier moyen pour solidariser la fiche avec la tige par insert de fixation.
La figure 4 représente un deuxième moyen pour solidariser la fiche avec la tige par collier de serrage.
La figure 5 représente une variante de réalisation de l'invention dans laquelle la tige pleine est constituée de deux demi-tiges longitudinales.
La figure 6 représente un collier d'union de deux tiges entre elles.

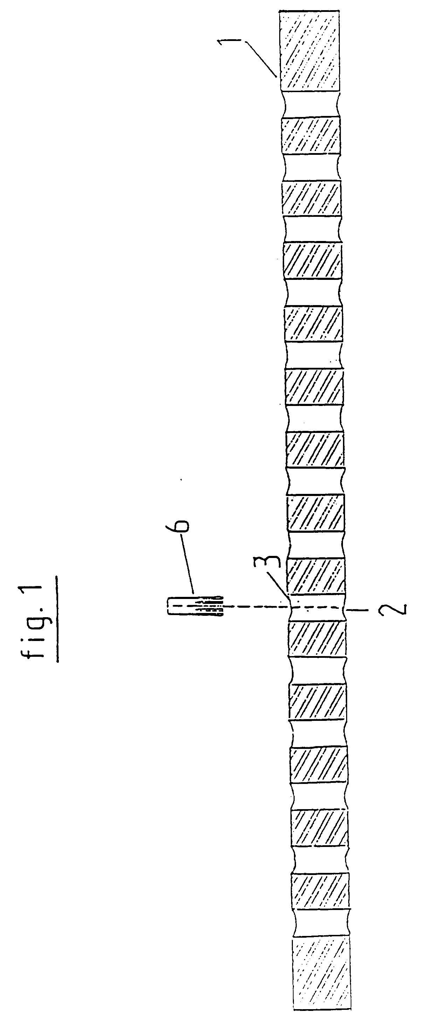

La figure 1 représente une vue en coupe d'une tige pleine présentant des orifices transversaux 2 régulièrement espacés sur toute la longueur de tige.

Ils présentent une ouverture tronconique 3 destinée à permettre le passage de la fiche munie d'un moyen pour la solidariser avec la tige, tel qu'un insert de fixation 6.

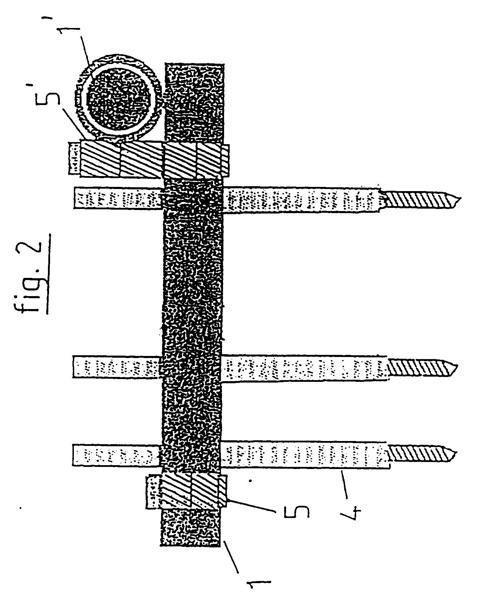

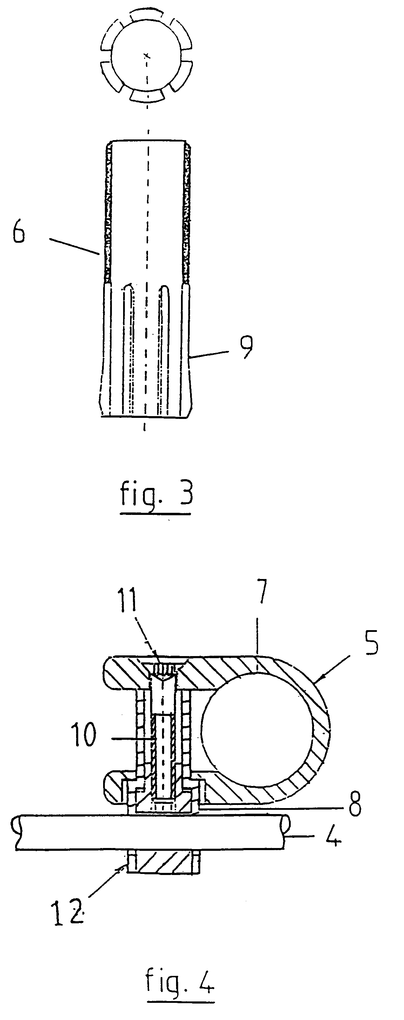

La figure 2 représente une tige pleine 1 munie de fiches 4 et de colliers de serrage 5 et 5' munis de moyens de blocage. Les fiches 4 sont engagées dans les logements cylindriques 2 de la tige 1. Elles sont maintenues en place par des moyens de fixation de deux types : des inserts de fixation 6 insérés dans les tiges et coaxiaux par rapport aux fiches tels que schématisés sur la figure 3 et des colliers de serrage 5 tels que schématisés sur la figure 4 qui sont des moyens supplémentaires de fixation des fiches, pour assurer une versatilité maximale du fixateur. Les inserts de fixation présentent sur leur demi longueur des lames 9 qui leur permettent d'être insérés en force dans les orifices 2 dans lesquels ils prennent place autour des fiches.

Comme il a été mentionné plus haut, l'opérateur peut faire appel à des moyens supplémentaires pour solidariser les fiches avec les tiges, tels que les colliers de serrage 5 de la figure 4 munis de moyens de blocage. Ces colliers 5 réalisés en matériau radiotransparent présentent de préférence une surface rugueuse afin d'adhérer à la tige. Ils ne peuvent pas glisser sur la tige et sont encliquetables. Ils présentent une bride 7 de serrage pour enserrer la tige 1 à laquelle est associée un manchon cylindrique de blocage 8, qui permet le blocage du collier par vissage. Le manchon 8 présente une partie taraudée 10 destinée à recevoir le filetage d'un boulon 11.

Le boulon 11 est tel qu'il est freiné dans son desserrage et comporte un blocage automatique.

L'utilisation du collier 5 assure donc au fixateur une versatilité d'implantation des fiches 4. En effet, dans certains cas de fractures il est nécessaire d'utiliser une fiche 4 déportée en l'inclinant par rapport à l'axe perpendiculaire à la tige. Le collier de serrage comprend à cet effet une deuxième bride 12 qui enserre la fiche 4 déportée.

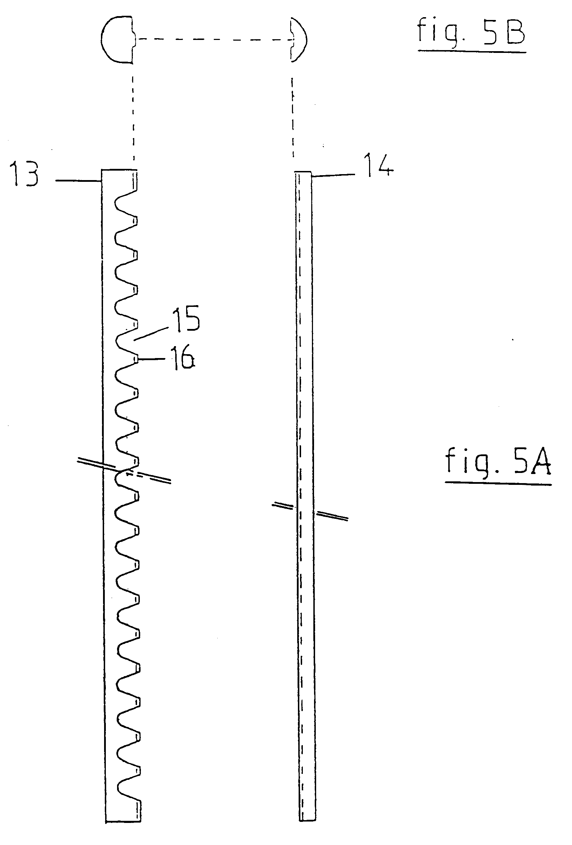

La figure 5A présente une variante de réalisation du fixateur selon l'invention. La tige pleine 1 est ici constituée de deux demi-tiges longitudinales dissemblables dont l'une d'elles 13 présente des évidements 15 régulièrement espacés, au fond arrondi. La réunion de ces deux demi-tiges forme une tige pleine entière crantée dont les crans ou logements sont destinés à recevoir les fiches 4.

Sur la figure 5B, la demi-tige 14 présente un évidement rectangulaire sur toute sa longueur, de telle manière que cet évidement puisse s'emboîter sur les parties saillantes 16 en regard de la demi-tige 13.

Ces deux demi-tiges 13 et 14 s'emboîtent parfaitement l'une dans l'autre et sont accolées par des colliers de serrage 5 tels que décrits précédemment.

La figure 2 ébauche un assemblage de deux tiges 1 et 1' à l'aide d'un collier d'union 5'.

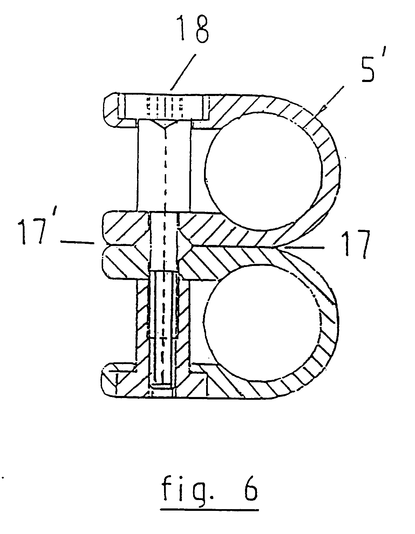

Ce collier d'union 5' représenté sur la figure 6 est constitué de deux colliers simples articulables autour de leur axe de serrage l'un par rapport à l'autre sur leurs surfaces communes 17 et 17' qui présentent la particularité d'être rugueuses.

Le boulon 18 a une longueur double de celui du collier simple.

Les colliers simples de ce collier d'union peuvent tourner l'un par rapport à l'autre autour de l'axe du boulon 18 avant serrage de celui-ci et sont bloqués par vissage dans l'orientation choisie. Le boulon 18 a la double fonction de solidariser les colliers avec les tiges et les colliers entre eux.

Le fixateur est prévu pour équiper des antennes chirurgicales mobiles et se présente sous blister après stérilisation par rayons gamma, ce qui permet de ne destériliser que les éléments utiles. Il est prévu pour un usage unique.

Le chirurgien guidé par la tige perforée qui sert de viseur à travers laquelle coulisse le perforateur de partie molle fait pénétrer dans la structure osseuse les fiches 4 autoforeuses et autotaraudeuses. Puis il met en place les différentes fiches en les faisant glisser dans les logements de la tige 1. Il procède ensuite au blocage des fiches selon les moyens décrits plus haut.

Selon le type de fracture, il peut être nécessaire d'utiliser un collier d'union 5' reunissant deux tiges pleines perforées présentant des orifices avec ouverture tronconique comme indiqué sur la figure 1 ou deux tiges constituées de deux demi-tiges 13 et 14 comme représentées sur la figure 5A ou encore deux tiges dont l'une est du type de celle représentée sur la figure 1 et l'autre du type de celle constituée par deux demi-tiges 13 et 14.

Selon le type de fracture, il peut être aussi nécessaire d'utiliser une fiche déportée en l'inclinant par rapport à l'axe perpendiculaire à la tige et dans ce cas la fiche est maintenue en place par un collier porte-fiche.

## Revendications

1. Appareil orthopédique linéaire de fixation externe, permettant de réduire une fracture et d'immobiliser un os fracturé, comportant au moins une tige rigide (1) en une seule pièce présentant des orifices transversaux régulièrement espacés sur toute la longueur de la tige, pour le passage d'une pluralité de fiches (4), munie de moyens pour la solidariser (6,5) avec ces fiches (4), caractérisé en ce que lesdites tiges sont pleines, et en ce que lesdits moyens pour solidariser les tiges (1) avec les fiches (4) sont des colliers de serrage (5) encliquetables, munis de moyens de blocage (8,11), ou des inserts (6) de fixation, coaxiaux par rapport aux fiches (4), les tiges (1), les fiches (4) et les moyens pour les solidariser (6,5) avec les fiches (4) étant en matériaux à la fois radiotransparents, amagnétiques et non conducteurs d'électricité.

2. Appareil orthopédique linéaire de fixation externe, selon la revendication 1, caractérisé en ce que le diamètre extérieur des inserts (6) de fixation est variable, pour s'adapter au diamètre des fiches (4).

3. Appareil orthopédique linéaire de fixation externe, selon la revendication 1, caractérisé en ce que les orifices transversaux de la tige pleine (1) présentent des ouvertures tronconiques (3).

4. Appareil orthopédique linéaire de fixation externe, selon la revendication 1, constitué de deux demi-tiges rigides pleines (13,14) longitudinales, caractérisé en ce que l'une seulement des demi-tiges présente des évidements (15) au fond arrondi, répartis régulièrement, définissant un crantage de la tige (1).

5. Appareil orthopédique linéaire de fixation externe, selon les revendications 1 et 4, caractérisé en ce que les deux demi-tiges longitudinales (13,14) constituant la tige (1) rigide pleine sont assemblées par au moins un collier (5) de serrage muni d'un moyen de blocage (8,11).

## Claims

1. A linear orthopaedic device for external setting, which makes it possible to set a fracture and to immobilise a fractured bone, comprising at least one rigid single-piece rod (1) having regularly spaced transverse holes over the entire length of the rod, for a plurality of plugs (4) to pass through, which is provided with means (6, 5) for connecting it to these plugs (4), characterised in that said rods are solid, and in that said means for connecting the rods (1) to the plugs (4) are snap-on clamping collars (5) provided with blocking means (8, 11), or fixing inserts (6), coaxial to the plugs (4), the rods (1), the plugs (4) and the means (6, 5) for connecting them to the plugs (4) being made of materials which are at the same time radiotransparent, non-magnetic and non-conductive of electricity.

2. A linear orthopaedic device for external setting according to Claim 1, characterised in that the external diameter of the fixing inserts (6) is variable, in order to adapt to the diameter of the plugs (4).

3. A linear orthopaedic device for external setting according to Claim 1, characterised in that the transverse orifices of the solid rod (1) have frustoconical openings (3).

4. A linear orthopaedic device for external setting according to Claim 1, formed of two rigid, solid longitudinal half-rods (13, 14), characterised in that only one of these half-rods has recesses (15) having a rounded base, distributed regularly, defining a serration of the rod (1).

5. A linear orthopaedic device for external setting according to Claims 1 and 4, characterised in that the two longitudinal half-rods (13, 14) forming the rigid, solid rod (1) are assembled by at least one clamping collar (5) provided with a blocking means (8, 11).

## Patentansprüche

1. Gerade orthophädische Vorrichtung zur externen Fixation, die es ermöglicht, bei Knochenbrüchen zu repositionieren und einen gebrochenen Knochen ruhigzustellen, umfassend zumindest einen starren, einstückigen Schaft (1), der über seine gesamte Länge gleichmäßig voneinander beabstandete Querlöcher aufweist, um eine Vielzahl Stifte (4) hindurchzuführen, ausgestattet mit Festmachmitteln (6,5), um ihn mit diesen Stiften (4) fest zu verbinden, dadurch gekennzeichnet, daß die Schäfte massiv sind und daß die Mittel zum festen Verbinden der Schäfte (1) mit den Stiften (4) mit Verriegelungsmitteln (8,11) versehene Schnapp-Klemmschellen (5) oder bezogen auf die Stifte (4) koaxiale Befestigungseinsätze (6) sind, wobei die Schäfte (1), die Stifte (4) und die Mittel (6,5), um die Schäfte fest mit den Stiften (4) zu verbinden, aus Materialien bestehen, die gleichzeitig strahlendurchlässig, nichtmagnetisch und nichtleitend sind.

2. Gerade orthophädische Vorrichtung zur externen Fixation nach Anspruch 1, dadurch gekennzeichnet, daß der Außendurchmesser der Befestigungseinsätze (6) variabel ist, sodaß er sich an den Durchmesser der Stifte (4) anpaßt.

3. Gerade orthopädische Vorrichtung zur externen Fixation nach Anspruch 1, dadurch gekennzeichnet, daß die Querlöcher des massiven Schafts (1) kegelstumpfförmige Öffnungen (3) darstellen.

4. Gerade orthopädische Vorrichtung zur externen Fixation nach Anspruch 1, die aus zwei länglichen massiven starren Halbschäften (13,14) besteht, dadurch gekennzeichnet, daß nur einer der Halbschäfte regelmäßig verteilte Vertiefungen (15) mit abgerundetem Boden aufweist, die eine Rastung des Schaftes (1) definieren.

5. Gerade orthopädische Vorrichtung zur externen Fixation nach Anspruch 1 und 4, dadurch gekennzeichnet, daß die beiden länglichen Halbschäfte (13,14), die den massiven starren Schaft (1) bilden, durch zumindest eine Klemmschelle (5) aneinandergefügt sind, die mit einem Verriegelungsmittel (8,11) versehen ist.
